# EUROPÄISCHE PATENTSCHRIFT

(11) **EP 0 998 483 B1**
(45) Veröffentlichungstag und Bekanntmachung des Hinweises auf die Patenterteilung: **13.06.2007**
(21) Anmeldenummer: 98941375.2
(22) Anmeldetag: 21.07.1998
(51) Int. Cl.: C07H 21/00

(54) **NUCLEINSÄUREMOLEKÜL, KIT UND VERWENDUNG**
NUCLEIC ACID MOLECULE, TEST KIT AND USE
MOLECULE D'ACIDE NUCLEIQUE, NECESSAIRE ET UTILISATION

(30) Priorität: 21.07.1997 DE 19731292
(43) Veröffentlichungstag der Anmeldung: 10.05.2000
(73) Patentinhaber: Biotecon Diagnostics GmbH, 10589 Berlin (DE)
(72) Erfinder: BERGHOF, Kornelia, D-12355 Berlin (DE); GASCH, Alexander, D-13359 Berlin (DE); MASON-BROWN, Charles, D-10245 Berlin (DE); WILBORN, Freimut, D-14057 Berlin (DE); ROLFS, Arndt, D-18055 Rostock (DE)
(74) Vertreter: Boeters, Hans Dietrich
(86) Internationale Anmeldenummer: PCT/EP1998/004510
(87) Internationale Veröffentlichungsnummer: WO 1999/005159

(56) Entgegenhaltungen:
- WO-A-90/14444
- WO-A-93/11264
- US-A- 5 582 975
- DATABASE EMBL(EMPRO) EMBL, Heidelberg Accession Number: L36472, 17. November 1994 C.J.GREEN ET AL.: "An unusual rRNA-tRNA gene organisation in S. aureus" XP002088806
- DATABASE EMBL(EMPRO) EMBL, Heidelberg Accession Number: L11530, 30. Juni 1993 GREEN C.J. ET AL. : "S. aureus transfer RNA sequence with two RNAs" XP002088803 & GREEN C.J. ET AL. : "S. aureus has clustered tRNA genes" J. BACTERIOL., Bd. 175, 1993, Seiten 5091-5096,
- DATABASE EMBL(EMPRO) EMBL, Heidelberg Accession Number: K01134, 13. Juni 1985 MACHATT M.A.: "A. punctata 23S ribosomal RNA" XP002088804
- DATABASE EMBL(EMPRO) EMBL, Heidelberg Accession Number: X68425, 29. März 1993 LUDWIG ET AL.: "S. aureus gene for 23S RNA" XP002088805 & LUDWIG ET AL.: "Complete 23S ribosomal RNA sequences of Gram-positive bacteria with a low DNA G+C content" SYST. APPL. MICROBIOL., Bd. 15, 1992, Seiten 487-501, XP002008633
- DATABASE WPI Week 9747 Derwent Publications Ltd., London, GB; AN 97-374922 XP002100941 & CA 2 194 411 A (HUMAN GENOME SCIENCES INC.), 6. Juli 1997

## Beschreibung

### Allgemeiner Hintergrund der Erfindung

Ein Bakterium von hoher klinischer Relevanz ist das gram-positive Bakterium *Staphylococcus aureus.* Es ist eines der häufigsten Verursacher von nosocomialen (im Krankenhaus übertragenen) Infektionen, deren Verbreitungen durch das Auftreten verschiedener Antibiotikaresistenzen (z.B. Resistenz gegen Methicillin und Vancomycin) schwer zu kontrollieren sind. *Staphylococcus aureus* gehört zudem zu den häufigsten Verursachern von Lebensmittelvergiftungen, welche meist durch Enterotoxine verursacht werden. Das häufige Vorkommen von *Staphylococcus aureus* in Lebensmittelprodukten erfordert daher eine regelmäßige Untersuchung potentiell kontaminierter Produkte. Konventionelle mikrobiologische Verfahren zum Nachweis von *Staphylococcus aureus* sind sehr zeitaufwendig (mindestens 4 Tage). Es besteht daher ein großer Bedarf nach alternativen Methoden, mit denen die Anwesenheit von *Staphylococcus aureus* im Verlauf des Produktionsprozesses (von den Rohstoffen bis hin zum fertigen Produkt) schnell und sicher diagnostiziert werden kann.

Für den routinemäßigen Einsatz zum Nachweis von Mikroorganismen sind in den letzten Jahren eine Reihe neuer Methoden entwickelt worden. Hierzu zählen immunologische Verfahren, die auf dem Einsatz polyvalenter oder monoklonaler Antikörper beruhen, und Verfahren, bei denen Nucleinsäure-Sonden zum Nachweis mittels Hybridisierung an keimspezifische Nucleinsäuren eingesetzt werden. Als weitere Methoden werden diejenigen Verfahren beschrieben, die auf einer spezifischen Nucleinsäure-Amplifikation basieren, mit oder ohne anschließende Bestätigungsreaktion durch Nucleinsäure-Hybridisierung. Eingesetzte Verfahren zur Amplifikation von Nucleinsäuren sind z.B. die Polymerase-Kettenreaktion (polymerase chain reaction, PCR) [US Patente 4,683,195; 4,683,202; und 4,965,188], die Ligase-Kettenreaktion [WO Veröffentlichung 89/09835], die "self-sustained sequence replication" [EP 329,822], das "transcription based amplification system" [EP 310,229] und das Qβ RNA-Replikase-System [US Patent 4,957,858].

Die genannten auf Nucleinsäuren basierenden Verfahren sind so sensitiv, daß, anders als bei konventionellen mikrobiologischen Verfahren, eine langwierige Anreicherung des nachzuweisenden Mikroorganismus aus der zu untersuchenden Probe entfällt. Eine Untersuchung auf An- oder Abwesenheit des jeweiligen Mikroorganismus ist daher bei Anwendung der genannten auf Nucleinsäuren basierenden Verfahren in der Regel innerhalb eines Arbeitstages abgeschlossen. Insbesondere wenn für den Nachweis mittels konventioneller Verfahren mehrere Tage bis Wochen benötigt werden, wird hierdurch eine erhebliche Zeitverkürzung erreicht.

In letzter Zeit wurden eine Reihe von Schnelltests entwickelt, mit denen die Anwesenheit von *Staphylococcus* aureus deutlich verkürzt werden kann. Zu diesen gehören Koagulase-Tests, verschiedene Agglutinations-Tests, TNase-Tests (Enzymaktivität, monoklonale Antikörper), DNA-Hybridisierungs-Tests und PCR-Tests [zur Übersicht siehe BRAKSTAD und MÆLAND, (1995), APMIS 103, 209-218]. Bezüglich Schnelligkeit und Spezifität sind PCR-Tests den anderen Verfahren überlegen. Die bisher beschriebenen PCR-Tests zum spezifischen Nachweis der Spezies *Staphylococcus aureus* basieren auf dem Gen für thermostabile Nuclease [*nuc,* Brakstad et al., (1992), J. Clin. Microbiol. 30, 1654-1660] bzw. auf einem für Methicillin-Resistenz essentiellen Regulatorgen (*femA*), welches spezifisch für *Staphylococcus aureus* ist [Ünal et al., (1992), J. Clin. Microbiol. 30, 1685-1691; Vannuffel et al., (1995), J. Clin. Microbiol., 33, 2864-2867]. Mit Hilfe dieser PCR-Systeme gelang der Nachweis aller untersuchten Spezies von *Staphylococcus aureus.* Unklar ist jedoch die Frage, ob mit diesen PCR-Systemen auch der Nachweis von Koagulase-negativen Stämmen der Spezies *Staphylcoccus aureus* möglich ist.

Ziel der hier dargestellten Erfindung war die Etablierung eines PCR-Systems, dessen Einsatz als Primer und/oder Sonden eine möglichst vollständige Erfassung aller Vertreter der Spezies *Staphylococcus aureus* sicherstellt.

Je nach Größe der zu detektierenden Gruppe von Mikroorganismen und der evolutionären Verwandtschaft von abzugrenzenden (nicht zu erfassenden) Mikroorganismen erfordert ein auf differentiellen DNA-Sequenzen basierender Nachweis sehr umfangreiche Vorarbeiten, um jeweils geeignete DNA-Sequenzen mit der gewünschten Spezifität zu finden. Die hier dargelegte Erfindung betrifft solche DNA-Sequenzen, mit denen der Schnellnachweis von *Staphylococcus aureus* möglich ist.

### Beschreibung der Erfindung

Die erfindung ist durch den Gegenstand des Ansprüchs definiert.

Zur Detektion von S. aureus mittels Nucleinsäure-Hybridisierung oder -Amplifikation werden die in den Ansprüchen definierten keimspezifischen Oligonucleotide eingesetzt. Eine Hybridisierung an DNA bzw. eine Amplifikation von DNA bei Einsatz dieser keimspezifischen Oligonucleotide (z.B. als Primer oder Sonden) mit den oben genannten Verfahren kann, unter geeigneten Reaktionsbedingungen, nur dann erfolgen, wenn die DNA der jeweils nachzuweisenden Mikroorganismen anwesend ist.

Prokaryontische Ribosomen beinhalten in der Regel drei distinkte Nucleinsäurekomponenten, welche allgemein als 5S, 16S und 23S rRNA (ribosomale Ribonucleinsäure) bekannt sind. Die genetische Information für diese Ribonucleinsäuren (rDNA) ist im Genom typischerweise in Form von Tandems angeordnet. Die typische Organisation einer solchen Einheit ist 16S-23S-5S, wobei die drei Gene durch kurze hypervariable intergenische Regionen voneinander getrennt sind. Die Einheiten sind im Genom mehrfach vorhanden, wobei die Anzahl der sich wiederholenden Einheiten in verschiedenen Bakterien variieren kann. Die hohe Konservierung der DNA-Sequenz im Bereich der 16S rDNA, der 23S rDNA und der 5S rDNA über das gesamte Bakterienreich ermöglicht ein Design von nicht-spezifischen Oligonucleotiden auch ohne genaue Kenntnis der DNA-Sequenzen der zu untersuchenden Mikroorganismen. Solche nicht-spezifischen Oligonucleotide sind charakteristisch für eine größere, in der Regel phylogenetisch verwandte Gruppe von Mikroorganismen. Durch Einsatz dieser nicht-spezifischen Oligonucleotide gelingt dem Fachmann, z.B. nach entsprechenden Vorversuchen durch DNA-Amplifikation mittels PCR, die Isolation von rDNA-Fragmenten, z.B. der 23S/5S intergenischen Region eines beliebigen Mikroorganismus. Durch DNA-Sequenzierung kann dann die Sequenz der hypervariablen intergenischen Regionen des betreffenden Mikroorganismus bestimmt werden.

DNA-Sequenzierung der 23S/5S intergenischen Region einer möglichst großen Anzahl nachzuweisender Bakterien (z.B. von verschiedenen *Staphylococcus*-Spezies) einerseits und nachfolgender Vergleich dieser DNA-Sequenzen andererseits erlauben das Auffinden von DNA-Bereichen, die in der untersuchten Gruppe (z.B. alle *Staphylococcus-*Spezies) nicht oder nur unwesentlich verändert sind.

DNA-Sequenzierung der 23S/5S intergenischen Region einer möglichst großen Anzahl von nicht-nachzuweisenden Bakterien (z.B allen Bakterien, die nicht zur Gattung *Staphylococcus* gehören) einerseits und nachfolgender Vergleich dieser DNA-Sequenzen mit der Sequenz der nachzuweisenden Bakterien (z.B. verschiedene *Staphylococcus*-Spezies) andererseits erlauben das Auffinden von DNA-Sequenzen, die für die nachzuweisenden Bakterien (z.B. alle *Staphylococcus*-Spezies) charakteristisch sind. Aus diesen DNA-Sequenzen können wiederum Oligonucleotide abgeleitetet werden, die als Primer und/oder Sonden in auf Nucleinsäuren basierenden Verfahren einsetzbar sind, mit dem Ziel, die jeweilige Gruppe von Bakterien (z.B. alle Spezies der Gattung *Staphylococcus)* spezifisch nachzuweisen.

Die in der vorliegenden Erfindung beschriebenen keimspezifischen Oligonucleotide zum Nachweis der Spezies *Staphylococcus* aureus, entsprechen der 235/55 intergenischen Region und dem direkt angrenzenden Bereich der 23S rDNA. Die DNA-Sequenz in dieser Region wurde für eine Vielzahl von Bakterien bestimmt. Nach exakten Sequenzvergleichen wurden keimspezifische Nucleinsäuresequenzen bestimmt, von denen Primer und/oder Sonden für einen Einsatz in einem Spezies-/Genus-spezifischen Nachweisverfahren abgeleitet werden können.

Die der Erfindung zugrundeliegende Aufgabe wird durch die in den Ansprüchen definierten kits gelöst.

Beschrieben ist auch ein Nucleinsäuremolekül, welches selektiv an RNA oder DNA einer Gruppe von Bakterien der Gattung *Staphylococcus* hybridisiert, und dadurch gekennzeichnet ist, daß es mindestens 10 aufeinanderfolgende Nucleotide des Bereiches zwischen -113 und +58 relativ zum 3'-Ende der 23S rDNA von *Staphylococcus aureus* (ATCC 6538) oder die zu ihnen komplementären Nucleotide beinhaltet.

Weiter beschrieben ist ein Nucleinsäuremolekül, welches selektiv an RNA oder DNA einer Gruppe von Bakterien der Gattung *Staphylococcus* hybridisiert, und dadurch gekennzeichnet ist, daß es mindestens 10 aufeinanderfolgende Nucleotide des Bereiches von
(i) Nucleotidposition 54 bis 83 von SEQ ID NO 1 oder
(ii) Nucleotidposition 100 bis 166 von SEQ ID NO 1 oder
(iii) den zu (i) oder (ii) komplementären Sequenzen beinhaltet.

Weiter beschrieben ist ein Nucleinsäuremolekül zum Nachweis der An- oder Abwesenheit von Bakterien, welche einer Gruppe von Bakterien der Gattung *Staphylococcus* angehören, und dadurch gekennzeichnet ist, daß es die Unterscheidung von nachzuweisenden Bakterien und nicht-nachzuweisenden Bakterien mittels Verfahren der Nucleinsäurehybridisierung und/oder Nucleinsäureamplifikation unter geeigneten Reaktionsbedingungen ermöglicht und diese Unterscheidung durch eine unterschiedliche Nucleinsäuresequenz der genomischen DNA und/oder RNA von nachzuweisenden gegenüber nicht-nachzuweisenden Bakterien an mindestens einer Basenposition im Bereich der SEQ ID NO: 1 oder der hierzu komplementären Sequenz möglich ist oder erleichtert wird.

Weitere beschrieben ist ein Nucleinsäuremolekül zum Nachweis der An- oder Abwesenheit von Bakterien, welche einer Gruppe von Bakterien der Gattung *Staphylococcus* angehören, und dadurch gekennzeichnet ist, daß es die Unterscheidung von nachzuweisenden Bakterien und nicht-nachzuweisenden Bakterien mittels Verfahren der Nucleinsäurehybridisierung und/oder Nucleinsäureamplifikation unter geeigneten oder an sich bekannten Reaktionsbedingungen ermöglicht und diese Unterscheidung durch eine unterschiedliche Nucleinsäuresequenz der genomischen DNA und/oder RNA von nachzuweisenden gegenüber nicht-nachzuweisenden Bakterien an mindestens einer Basenposition in
(i) dem Bereich 54 bis 83 von SEQ ID NO 1 oder
(ii) dem Bereich 100 bis 166 von SEQ ID NO 1 oder
(iii) der zu (i) oder (ii) komplementären Sequenz möglich ist oder erleichtert wird.

Weiter beschrieben ist ein Nucleinsäuremolekül der SEQ ID NO 1 oder der hierzu komplementären Sequenz, insbesondere für den erfindungsgemäßen Nachweis.

Weiter beschrieben ist ein Nucleinsäuremolekül mit gegenüber einem Nucleinsäuremolekül gemäß SEQ ID NO 1 verkürzter Sequenz, und zwar
(i) einer Sequenz des Bereichs oder im Bereich der Nucleotidpositionen 54 bis 83 oder
(ii) einer Sequenz des Bereichs oder im Bereich der Nucleotidpositionen 100 bis 166 oder
(iii) einer Sequenz, die zu einer Sequenz gemäß (i) oder (ii) komplementär ist.

Weiter beschrieben ist ein Nucleinsäuremolekül mit gegenüber einem Nucleinsäuremolekül gemäß SEQ ID NO 1 verkürzter Sequenz, nämlich
(i) der SEQ ID NO 2 oder
(ii) der SEQ ID NO 3 oder
(iii) der SEQ ID NO 4 oder
(iv) der zu (i), (ii) und (iii) jeweils komplementären Sequenz.

Weiter beschrieben ist ein weiteres bzw. anderes Nucleinsäuremolekül, das dadurch gekennzeichnet ist, daß es hinsichtlich seiner Sequenz bei mindestens 10 aufeinanderfolgenden Nucleotiden seiner Nucleotidkette
(i) mit einem Nucleinsäuremolekül gemäß einem der vorhergehenden Ansprüche identisch ist oder
(ii) in 9 von 10 aufeinanderfolgenden Nucleotiden mit einem Nucleinsäuremolekül gemäß einem der vorhergehenden Ansprü che übereinstimmt oder
(iii) in 8 von 10 aufeinanderfolgenden Nucleotiden mit einem Nucleinsäuremolekül gemäß einem der vorhergehenden Ansprü che übereinstimmt oder
(iv) zu mindestens 90 % mit einem Nucleinsäuremolekül gemäß ei nem der vorhergehenden Ansprüche homolog ist.

Ein derartiges Nucleinsäuremolekül kann dadurch gekennzeichnet sein, daß es 10 bis 250 und vorzugsweise 15 bis 30 Nucleotide lang ist.

Ein Beispiel für ein Nucleinsäuremolekül gemäß (i) ist durch die SEQ ID NO 5 gekennzeichnet.

Ein beschriebenes Nucleinsäuremolekül kann dadurch gekennzeichnet sein, daß es einzelsträngig oder doppelsträngig vorliegt.

Ein beschriebenes Nucleinsäuremolekül kann dadurch gekennzeichnet sein, daß es
(i) als DNA oder
(ii) als (i) entsprechende RNA oder
(iii) als PNA (siehe im folgenden) vorliegt,
wobei das Nucleinsäuremolekül gegebenenfalls in einer für analytische Nachweisverfahren, insbesondere auf Basis von Hybridisierung und/oder Amplifizierung, an sich bekannten Weise modifiziert ist.

Ein derartiges Nucleinsäuremolekül kann dadurch gekennzeichnet sein, daß das Nucleinsäuremolekül dadurch modifiziert ist, daß bis zu 10 % der Nucleotide, insbesondere 1 oder 2 Nucleotide, durch für Sonden und/oder Primer an sich bekannte analoge Bausteine ersetzt sind, insbesondere durch Nucleotide, die bei Bakterien nicht natürlich vorkommen.

Ein derartiges Nucleinsäuremolekül kann dadurch gekennzeichnet sein, daß das Nucleinsäuremolekül dadurch oder zusätzlich dadurch modifiziert bzw. markiert ist, daß es in einer für analytische Nachweisverfahren an sich bekannten Weise ein oder mehrere radioaktive Gruppen, farbige Gruppen, fluoreszierende Gruppen, Gruppen zur Immobilisierung an fester Phase und/oder Gruppen für eine indirekte oder direkte Reaktion, insbesondere eine enzymatische Reaktion, aufweist, insbesondere mit Hilfe von Antikörpern, Antigenen, Enzymen und/oder Substanzen mit Affinität zu Enzymen oder Enzymkomplexen.

Die Erfindung betrifft einen in den Ansprüchen definierten Kit gekennzeichnet durch die in den Ansprüchen definierten Nucleinsäuremoleküle.

Eine weitere Ausführungsform der Erfindung betrifft eine Verwendung eines erfindungsgemäßen Kits zum Nachweis der An-oder Abwesenheit von einer Gruppe von Bakterien der Gattung *Staphylococcus* angehören.

Diese Verwendung ist dadurch gekennzeichnet sein, daß es sich bei der Gruppe von Bakterien der Gattung *Staphylococcus* ausschließlich um *Staphylococcus aureus-*Stämme handelt.

Diese Verwendungen können dadurch gekennzeichnet sind, daß man eine Nucleinsäurehybridisierung und/oder eine Nucleinsäureamplifikation durchführt.

Diese Verwendung kann dadurch gekennzeichnet sein, daß man eine Polymerase-Kettenreaktion als Nucleinsäureamplifikation durchführt.

Diese Verwendung ist dadurch gekennzeichnet sein, daß man anhand von Unterschieden im Bereich eines Nucleinsäuremoleküls der SEQ ID NO 1 unterscheidet.

Zum Nachweis der besagten Gruppe von Mikroorganismen werden Nucleinsäuren, vorzugsweise genomische DNA, zunächst aus den in einer zu untersuchenden Probe bzw. Bakterienkultur enthaltenen Zellen freigesetzt. Mittels Nucleinsäurehybridisierung kann dann, und zwar unter Einsatz der erfindungsgemäßen keimspezifischen Oligonucleotide als Sonde, der direkte Nachweis von keimspezifischen Nucleinsäuresequenzen in der zu untersuchenden Probe erfolgen. Geeignet hierzu sind verschiedene dem Fachmann bekannte Verfahren, wie z.B. "Southern blot" oder "dot blot".

Bevorzugt ist jedoch, vor allem wegen der höheren Empfindlichkeit, ein indirektes Nachweisverfahren, bei dem die gesuchten DNA/RNA-Sequenzen zunächst mittels der o.g. Verfahren zur Amplifikation von Nucleinsäuren, vorzugsweise PCR, amplifiziert werden.

Die Amplifikation von freigesetzter DNA/RNA unter Verwendung der genannten Verfahren kann unter Einsatz von keimspezifischen Oligonucleotiden als Primer erfolgen. Dabei werden nur in dem Fall spezifische Amplifikate gebildet, in dem DNA/RNA des nachzuweisenden Mikroorganismus anwesend ist. Durch eine nachgeschaltete Detektionsreaktion unter Verwendung von keimspezifischen Oligonucleotiden als Sonden kann die Spezifität des Nachweisverfahrens erhöht werden. Für diese nachgeschaltete Detektionsreaktion ist ebenso die Verwendung von nicht-keimspezifischen Oligonucleotiden als Sonden möglich.

Alternativ kann die Nucleinsäure-Amplifikation auch in Anwesenheit eines oder mehrerer nicht-spezifischer Oligonucleotide durchgeführt werden, so daß möglicherweise auch DNA/RNA anderer, nicht-nachzuweisender Mikroorganismen amplifiziert werden kann. Ein derartiges Amplifikationsverfahren ist in der Regel weniger spezifisch und sollte daher durch eine nachfolgende Detektionsreaktion mit einem oder mehreren keimspezifischen Oligonucleotid(en) als Sonde(n) abgesichert werden.

Dem Fachmann sind verschiedene Verfahren bekannt, mit denen die bei den indirekten Verfahren entstehenden Amplifikationsprodukte nachgewiesen werden können. Dazu gehören u.A. die Visualisierung mittels Gelelektrophorese, die Hybridisierung von Sonden an immobilisierte Reaktionsprodukte [gekoppelt an Nylon- oder Nitrocellulose-Filter ("Southern blots") oder z.B. an "beads" oder Mikrotiterplatten] und die Hybridisierung der Amplifikationsprodukte an immobilisierte Sonden (z.B. "reverse dot blots" oder mit Sonden gekoppelte "beads" oder Mikrotiterplatten).

Es sind eine Vielzahl verschiedener Varianten beschrieben, mit denen keimspezifische Oligonucleotide (z.B. Sonden und Primer) für die beschriebenen direkten oder indirekten Nachweisverfahren markiert bzw. modifiziert werden können. So können diese beispielsweise radioaktive, farbige, fluoreszierende oder anderweitig modifizierte bzw. modifizierende Gruppen enthalten, beispielsweise Antikörper, Antigene, Enzyme bzw. andere Substanzen mit Affinität zu Enzymen oder Enzymkomplexen. Sonden bzw. Primer können entweder natürlich vorkommende oder synthetisch hergestellte doppelsträngige oder einzelsträngige DNA oder RNA sein bzw. modifizierte Formen von DNA oder RNA, wie z.B. PNA (bei diesen Molekülen sind die Zucker-Einheiten durch Aminosäuren oder Peptide ausgetauscht). Einzelne oder mehrere Nucleotide der Sonden oder Primer können gegen analoge Bausteine (wie z.B. Nucleotide, die in der Ziel-Nucleinsäure nicht natürlich vorkommen) ersetzt sein. Bei den o.g. indirekten Nachweisverfahren kann Detektion auch über ein intern-markiertes Amplifikat geführt werden. Dies kann z. B. über den Einbau von modifizierten (z.B. Digoxigenin- oder Fluorescein-gekoppelten) Nucleosidtriphosphaten während der Amplifikationsreaktion erfolgen.

Die DNA-Sequenz von *Staphylococcus aureus* (ATCC 6538) im Bereich der 23S rDNA (1-113) und der 23S/5S intergenischen Region (114-171) lautet:

(Sequenz 1 = SEQ ID NO 1)
TTTCCCAACTTCGGTTATAAGATCCCTCAAAGATGATGAGGTTAATAGGTTCGAGGTGGAAGCA
TGGTGACATGTGGAGCTGACGAATACTAATCGATCGAAGACTTAATCAAAATAAATGTTTTGCG
AAGCAAAATCACTTTTACTTACTATCTAGTTTTGAATGTATAA

Die Sequenz im Bereich der 23S/5S-intergenischen Region wurde für 8 *Staphylococcus aureus*-Stämme sowie für mindestens je einen Vertreter folgender Spezies bestimmt: *Staphylococcus auricularis, Staphylococcus capitis, Staphylococcus cohnii, Staphylococcus epidermidis, Staphylococcus haemolyticus, Staphylococcus hominis, Staphylococcus saccharolyticus, Staphylococcus saprophyticus, Staphylococcus simulans, Staphylococcus warneri, Staphylococcus xylosus.* Die Sequenzvergleiche ergaben, daß die Sequenzen der verschiedenen Spezies in diesem Bereich einen hohen Homologiegrad aufweisen. Es lassen sich jedoch 3 Bereiche mit hoher Sequenzvariabilität erkennen, und zwar zwischen den Nucleotidpositionen 64 und 73, 110 und 121, sowie 127 und 156. Aus den Nucleotidsequenzen der verschiedenen *Staphylococcus-*Spezies in den Bereichen 54 bis 83 und 100 bis 166 lassen sich daher für den Nachweis von Bakterien der Gattung *Staphylococcus* geeignete Oligonucleotide ableiten. So können von Sequenz 1 mehrere Oligonucleotide abgeleitet werden, die die variablen Nucleotidpositionen 64 bis 73, 110 bis 121 und 127 bis 156 ganz oder teilweise überspannen und die sich als Primer oder Sonden für den selektiven Nachweis von Bakterien der Spezies Staphylo*coccus aureus* eignen.

Von Sequenz 1 wurden folgende als Primer für die PCR (Sequenz 2 und Sequenz 3) bzw. als Sonde (Sequenz 4 und Sequenz 5) besonders geeignete Oligonucleotide abgeleitet:
Oligonucleotid Sa1: (Sequenz 2 = SEQ ID NO 2) 5'-GTGGAAGCAT GGTGACAT-3'
Oligonucleotid Sa2: (Sequenz 3 = SEQ ID NO 3) 5'-TAAGTAAAAG TGATTTTGCT TCG-3'
Oligonucleotid Sa3: (Sequenz 4 = SEQ ID NO 4) 5'-CATTTATTTT GATTAAGTCT-3'
Oligonucleotid Sa4: (Sequenz 5 = SEQ ID NO 5) 5'-CATTTAAATT TGATTAAGTC-3'

### Beispiel 1: Nachweis von Bakterien der Spezies Staphylococcus aureus mit der Polymerase-Kettenreaktion

Aus Reinkulturen der in Tabelle 1 aufgeführten Bakterien wurde DNA mittels Standardverfahren isoliert. Je ca. 10 bis 100 ng dieser DNA-Präparationen wurde dann in Gegenwart von je 0,6 µM Oligonucleotid Sa1 (SEQ ID NO 2) und Sa2 (SEQ ID NO 3), 200 µM dNTP's (N = A/C/G/T-Mixtur; Boehringer Mannheim), 2 mM MgCl₂, 16 mM (NH₄)₂SO₄, 67 mM Tris/HCl (pH 8,8), 0,01 % Tween 20 und 0,03 U/µl Taq-Polymerase (Biomaster) in die PCR eingesetzt. Die PCR wurde in einem Perkin-Elmer 9600 Thermocycler mit dem nachfolgend aufgeführten Thermoprofil durchgeführt:

| | | |
|---|---|---|
| - initiale Denaturierung | 94 °C | 3 min |
| - Amplifikation (35 Zyklen) | 92°C | 30 sek |
| | 60°C | 10 sek |
| - finale Synthese | 72 °C | 5 min |

Nach Beendigung der PCR-Reaktion wurden die Amplifikationsprodukte mittels Agarose-Gelelektrophorese aufgetrennt und durch Anfärbung mit Ethidiumbromid visualisiert. Das erwartete Produkt von 94 bp Länge wurde nur in den Fällen beobachtet, in denen DNA von Stämmen der Spezies *Staphylococcus aureus* anwesend war (vergleiche **Tabelle 1**), nicht aber bei Anwesenheit von DNA der anderen getesteten Bakterien. Nach Beendigung des Laufes wurde die in den Gelen enthaltene DNA mittels Standard-Methoden auf Nylon-Filter transferiert und zur Überprüfung der Spezifität mit einer äquimolaren Mischung der am 5'-Ende biotinylierten Oligonucleotide Sa3 (SEQ ID NO 4) und Sa4 (SEQ ID NO 5) hybridisiert. Die Hybridisierung erfolgte in 5 x SSC , 2 % Blocking Reagenz, 0,1 % Laurylsarcosin, 0,02 % SDS und 20 pmol/ml Sonde für 4 h bei 43 °C. Gewaschen wurde in 2 x SSC, 0,1 % SDS für 1 x 5 min bei Raumtemperatur und 1 x 5 min bei 43 °C. Die Detektion erfolgte nach Standard-Methoden mittels alkalischer Phosphatase-Konjugaten (Extravidin, Fa SIGMA, # E-2636) in Anwesenheit von 5-Brom-4-chlor-3-indolylphosphat und 4-Nitro-Blue Tetrazoliumchlorid (Fa. Boehringer Mannheim).

Auf den Filtern wurde nur in den Fällen eine Bande beobachtet, in denen zuvor auch eine Bande auf dem Agarose-Gel sichtbar war (siehe **Tabelle 1**). Somit wurde sowohl mittels PCR wie auch mittels Hybridisierung die Anwesenheit sämtlicher der 50 getesteten *Staphylococcus* aureus-Stämme nachgewiesen. Unter den insgesamt 50 getesteten *Staphylococcus* aureus-Stämmen sind 2 Koagulase-negativ (BioteCon 7030 und BioteCon 7044), welche ebenfalls mit dem PCR-Verfahren erfasst werden. Hingegen wurde keiner der getesteten, nicht zu dieser Spezies gehörenden Bakterienstämme mit diesem System erfaßt.

**Tabelle 1: Resultate der PCR-Amplifikation mit den Oligonucleotiden Sa1 und Sa2 (SEQ ID NO 2 und SEQ ID NO 3) und nachfolgender Hybridisierung mit den Oligonucleotiden Sa3/Sa4 (SEQ ID NO 4 und SEQ ID NO 5)**

| **Spezies** | **Stammbezeichnung** | **PCR** | **Hybridisierung** |
|---|---|---|---|
| *Staphylococcus aureus* | ATCC 6538 | + | + |
| *Staphylococcus aureus* | BioteCon 194 | + | + |
| *Staphylococcus aureus* | BioteCon 195 | + | + |
| *Staphylococcus aureus* | BioteCon 196 | + | + |
| *Staphylococcus aureus* | BioteCon 197 | + | + |
| *Staphylococcus aureus* | BioteCon 494 | + | + |
| *Staphylococcus aureus* | BioteCon 514 | + | + |
| *Staphylococcus aureus* | BioteCon 528 | + | + |
| *Staphylococcus aureus* | BioteCon 529 | + | + |
| *Staphylococcus aureus* | BioteCon 530 | + | + |
| *staphylococcus aureus* | BioteCon 531 | + | + |
| *Staphylococcus aureus* | BioteCon 532 | + | + |
| *Staphylococcus aureus* | BioteCon 533 | + | + |
| *Staphylococcus aureus* | BioteCon 534 | + | + |
| *Staphylococcus aureus* | BioteCon 535 | + | + |
| *Staphylococcus aureus* | BioteCon 4286 | + | + |
| *Staphylococcus aureus* | BioteCon 4287 | + | + |
| *Staphylococcus aureus* | BioteCon 4288 | + | + |
| *Staphylococcus aureus* | BioteCon 4289 | + | + |
| *Staphylococcus aureus* | ATCC 25923 | + | + |
| *Staphylococcus aureus* | BioteCon 7010 | + | + |
| *Staphylococcus aureus* | BioteCon 7011 | + | + |
| *Staphylococcus aureus* | BioteCon 7012 | + | + |
| *Staphylococcus aureus* | BioteCon 7013 | + | + |
| *Staphylococcus aureus* | BioteCon 7014 | + | + |
| *Staphylococcus aureus* | BioteCon 7015 | + | + |
| *Staphylococcus aureus* | BioteCon 7016 | + | + |
| *Staphylococcus aureus* | BioteCon 7017 | + | + |
| *Staphylococcus aureus* | BioteCon 7018 | + | + |
| *Staphylococcus aureus* | BioteCon 7019 | + | + |
| *Staphylococcus aureus* | BioteCon 7020 | + | + |
| *Staphylococcus aureus* | BioteCon 7021 | + | + |
| *Staphylococcus aureus* | BioteCon 7022 | + | + |
| *Staphylococcus aureus* | BioteCon 7024 | + | + |
| *Staphylococcus aureus* | BioteCon 7025 | + | + |
| *Staphylococcus aureus* | BioteCon 7027 | + | + |
| *Staphylococcus aureus* | BioteCon 7028 | + | + |
| *Staphylococcus aureus* | BioteCon 7029 | + | + |
| *Staphylococcus aureus* | BioteCon 7030 | + | + |
| *Staphylococcus aureus* | BioteCon 7031 | + | + |
| *Staphylococcus aureus* | BioteCon 7032 | + | + |
| *Staphylococcus aureus* | BioteCon 7034 | + | + |
| *Staphylococcus aureus* | BioteCon 7035 | + | + |
| *Staphylococcus aureus* | BioteCon 7036 | + | + |
| *Staphylococcus aureus* | BioteCon 7039 | + | + |
| *Staphylococcus aureus* | BioteCon 7040 | + | + |
| *Staphylococcus aureus* | BioteCon 7041 | + | + |
| *Staphylococcus aureus* | BioteCon 7042 | + | + |
| *Staphylococcus aureus* | BioteCon 7043 | + | + |
| *Staphylococcus aureus* | BioteCon 7044 | + | + |
| *Staphylococcus arlettae* | DSM 20672 | - | - |
| *Staphylococcus auricularis* | DSM 20609 | - | - |
| *Staphylococcus capitis* subsp. *ureolyticus* | BioteCon 2687 | - | - |
| *Staphylococcus caprae* | DSM 20608 | - | - |
| *Staphylococcus carnosus* | DSM 20501 | - | - |
| Staphylococcus *caseolyticus* | DSM 20597 | - | - |
| *Staphylococcus chromogenes* | DSM 20454 | - | - |
| *Staphylococcus cohnii subsp. cohnii,* | DSM 20260 | - | - |
| *Staphylococcus delphini* | DSM 20771 | - | - |
| *Staphylococcus epidermidis* | BioteCon 515 | - | - |
| *Staphylococcus equorum* | DSM 20674 | - | - |
| *Staphylococcus gallinarum* | DSM 20610 | - | - |
| *Staphylococcus haemolyticus* | BioteCon 2847 | - | - |
| *Staphylococcus hominis* | DSM 20328 | - | - |
| *Staphylococcus hyicus* | DSM 20459 | - | - |
| *Staphylococcus intermedius* | DSM 20373 | - | - |
| *Staphylococcus kloosii* | DSM 20676 | - | - |
| *Staphylococcus lentus* | DSM 6672 | - | - |
| *Staphylococcus lugdunensis* | BioteCon 2681 | - | - |
| *Staphylococcus muscae* | DSM 7068 | - | - |
| *Staphylococcus saccharolyticus* | DSM 20359 | - | - |
| *Staphylococcus saprophyticus* | BioteCon 2685 | - | - |
| *Staphylococcus schleiferi subsp. schleiferi* | DSM 4808 | - | - |
| *Staphylococcus sciuri* | DSM 6671 | - | - |
| *Staphylococcus simulans* | DSM 20322 | - | - |
| *Staphylococcus warneri* | DSM 20036 | - | - |
| *Staphylococcus xylosus* | BioteCon 2683 | - | - |
| *Bacillus cereus* | DSM 31 | - | n.d. |
| Bacillus *coagulans* | DSM 1 | - | n.d. |
| Bacillus *brevis* | DSM 30 | - | n.d. |
| *Bacillus megaterium* | DSM 32 | - | n.d. |
| *Bacillus thuringiensis* | DSM 350 | - | n.d. |
| Bacillus *badius* | DSM 23 | - | n.d. |
| *Bacillus sphaericus* | BioteCon 3136 | - | n.d. |
| Bacillus *subtilis* | ATCC 6633 | - | n.d. |
| *Bacillus circulans* | BioteCon 4926 | - | n.d. |
| Bacillus *polymyxa* | ATCC 8523 | - | n.d. |
| *Bacillus mycoides* | BioteCon 4928 | - | n.d. |
| *Bacillus stearothermophilus* | DSM 456 | - | n.d. |
| *Pseudomonas aeruginosa* | BiotCon 682 | - | n.d. |
| *Pseudomonas fluorescens* | BioteCon 2439 | - | n.d. |
| *Pseudomonas cepacia* | BioteCon 672 | - | n.d. |
| *Pseudomonas chlororaphis* | BioteCon 1753 | - | n.d. |
| *Pseudomonas citronellolis* | DSM 50332 | - | n.d. |
| *Pseudomonas mendocina* | DSM 50017 | - | n.d. |
| *Pseudomonas pickettii* | BioteCon 3323 | - | n.d. |
| *Pseudomonas fragi* | DSM 3456 | - | n.d. |
| *Pseudomonas hydrophila* | BioteCon 4883 | - | n.d. |
| *Pseudomonas putida* | BioteCon 4884 | - | n.d. |
| *Pseudomonas oleovorans* | DSM 1045 | - | n.d. |
| *Pseudomonas pseudoalcaligenes* | DSM 50188 | - | n.d. |
| *Pseudomonas syringae* | DSM 10604 | - | n.d. |
| *Pseudomonas mendocina* | DSM 50017 | - | n.d. |
| *Pseudomonas corrugata* | DSM 7228 | - | n.d. |
| *Lactobacillus brevis* | DSM 1267 | - | n.d. |
| *Lactobacillus viridescens* | BioteCon 2592 | - | n.d. |
| *Lactobacillus kefiranofaciens* | DSM 5016 | - | n.d. |
| *Lactobacillus lindneri* | BioteCon 2599 | - | n.d. |
| *Lactobacillus fructivorans* | BioteCon 2598 | - | n.d. |
| *Lactobacillus casei* | DSM 20011 | - | n.d. |
| *Lactobacillus vaginalis* | DSM 5837 | - | n.d. |
| *Lactobacillus fermentum* | BioteCon 2597 | - | n.d. |
| *Lactobacillus intestinalis* | DSM 6629 | - | n.d. |
| *Lactobacillus bifermentans* | DSM 20003 | - | n.d. |
| *Lactobacillus curvatus* | DSM 20019 | - | n.d. |
| *Lactobacillus coryniformis subsp. torquens* | DSM 20004 | - | n.d. |
| *Lactobacillus acidophilus* | BioteCon 786 | - | n.d. |
| *Lactobacillus alimentarius* | DSM 20249 | - | n.d. |
| *Lactobacillus fructosus* | DSM 20349 | - | n.d. |
| *Lactobacillus malefermentans* | DSM 20177 | - | n.d. |
| *Lactobacillus kefir* | DSM 20485 | - | n.d. |
| *Lactobacillus salivarius subsp. salivarius* | DSM 20492 | - | n.d. |
| *Lactobacillus homohiochii* | DSM 20571 | - | n.d. |
| *Lactobacillus sanfrancisco* | DSM 20663 | - | n.d. |
| *Lactobacillus aviarius* | DSM 20655 | - | n.d. |
| *Lactobacillus ruminis* | DSM 20403 | - | n.d. |
| *Leuconostoc mesenteriodes subsp. mesenteriodes* | DSM 20240 | - | n.d. |
| *Leuconostoc mesenteriodes subsp. dextranicum* | DSM 20071 | - | n.d. |
| *Leuconostoc mesenteriodes subsp. cremoris* | DSM 20346 | - | n.d. |
| *Leuconostoc lactis* | DSM 20202 | - | n.d. |
| *Leuconostoc oenos* | DSM 20255 | - | n.d. |
| *Leuconostoc gelidum* | DSM 5578 | - | n.d. |
| *Leuconostoc carnosum* | DSM 5576 | - | n.d. |
| *Leuconostoc* citreum | DSM 20188 | - | n.d. |
| *Leuconostoc paramesenteroides* | DSM 20288 | - | n.d. |
| *Leuconostoc fallax* | DSM 20189 | - | n.d. |
| *Leuconostoc pseudomesenteroides* | DSM 5625 | - | n.d. |
| *Streptococcus downei* | DSM 5635 | - | n.d. |
| *Streptococcus sp.* | DSM 6176 | - | n.d. |
| *Streptococcus parauberis* | DSM 6631 | - | n.d. |
| *Streptococcus gordonii* | DSM 6777 | - | n.d. |
| *Streptococcus sp.* | DSM 20061 | - | n.d. |
| *Streptococcus equinus* | DSM 20062 | - | n.d. |
| *Streptococcus salivarius subsp. thermophilus* | DSM 20259 | - | n.d. |
| *Streptococcus canis* | DSM 20386 | - | n.d. |
| *Streptococcus cricetus* | DSM 20562 | - | n.d. |
| *Streptococcus anginosus* | DSM 20563 | - | n.d. |
| *Streptococcus rattus* | DSM 20564 | - | n.d. |
| *Streptococcus pneumoniae* | DSM 20566 | - | n.d. |
| *Streptococcus pleomorphus* | DSM 20574 | - | n.d. |
| *Streptococcus iniae* | DSM 20576 | - | n.d. |
| *Streptococcus hansenii* | DSM 20583 | - | n.d. |
| *Streptococcus ferus* | DSM 20646 | - | n.d. |
| *Streptococcus alactolyticus* | DSM 20728 | - | n.d. |
| *Streptococcus hyointestinalls* | DSM 20770 | - | n.d. |
| *Pediococcus parvulus* | DSM 20332 | - | n.d. |
| *Campylobacter jejuni subsp. jejuni* | DSM 4688 | - | n.d. |
| *Citrobacter freundii* | DSM 30040 | - | n.d. |
| *Shigella flexneri* | DSM 4782 | - | n.d. |
| *Pectinatus cerevisiiphilus* | DSM 2834 | - | n.d. |
| *Pediococcus inopinatus* | DSM 20285 | - | n.d. |
| *Proteus mirabilis* | BioteCon 4701 | - | n.d. |
| *Megasphaera cerevisiae* | DSM 20461 | - | n.d. |
| *Escherichia coli* | BioteCon 4949 | - | n.d. |

| | | | |
|---|---|---|---|
| n.d.: Die Hybridisierung wurde nicht durchgeführt | | | |

## Patentansprüche

1. Kit mit mehreren Oligonucleotiden als Primer und/oder Sonde für den analytischen Nachweis von Staphylococcus aureus-Stämmen, **gekennzeichnet durch**
(a) ein Oligonucleotid, das mindestens 10 aufeinanderfolgende Nucleotide von SEQ ID NO 1 oder die zu ihnen komplementären Nucleotide beinhaltet, sowie
(b) ein Oligonucleotid, das mindestens 10 aufeinanderfolgende Nucleotide des Bereichs von
(i) Nucleotidposition 100 bis 166 von SEQ ID NO 1 oder
(ii) die zu (i) komplementäre Sequenz beinhaltet.

2. Kit nach Anspruch 1 mit einem Oligonucleotid gemäß (a) mit gegenüber SEQ ID NO 1 verkürzter Sequenz und zwar
(i) einer Sequenz des Bereichs oder im Bereich der Nucleotidpositionen 54 bis 83 oder
(ii) einer Sequenz des Bereichs der Nucleotidpositionen 110 bis 121 oder
(iii) einer Sequenz des Bereichs der Nucleotidpositionen 127 bis 156 oder
(iv) einer Sequenz, die zu einer Sequenz gemäß (i), (ii) oder (iii) komplementär ist.

3. Kit nach Anspruch 1 mit einem Oligonucleotid mit gegenüber SEQ ID NO 1 verkürzter Sequenz, nämlich
(i) der SEQ ID NO 2 oder
(ii) der SEQ ID NO 3 oder
(iii) der SEQ ID NO 4 oder
(iv) der zu (i), (ii) und (iii) jeweils komplementären Sequenz.

4. Kit nach Anspruch 1 mit einem Oligonucleotid, das **dadurch *gekennzeichnet* ist, daß** es hinsichtlich seiner Sequenz bei mindestens 10 aufeinanderfolgenden Nucleotiden seiner Nucleotidkette
(i) mit einem Oligonucleotid gemäß einem der vorhergehenden Ansprüche 2 oder 3 identisch ist oder
(ii) in 9 von 10 aufeinanderfolgenden Nucleotiden mit einem Oligonucleotid gemäß einem der vorhergehenden Ansprüche 2 oder 3 übereinstimmt oder
(iii) in 8 von 10 aufeinanderfolgenden Nucleotiden mit einem Oligonucleotid gemäß einem der vorhergehenden Ansprüche 2 oder 3 übereinstimmt.

5. Kit nach Anspruch 4, **dadurch *gekennzeichnet*, daß** das Oligonucleotid 10 bis 250 und vorzugsweise 15 bis 30 Nucleotide lang ist, insbesondere **dadurch *gekennzeichnet*, daß** es das Nucleinsäuremolekül mit der Sequenz SEQ ID NO 5 ist.

6. Kit nach einem der vorhergehenden Ansprüche, **dadurch *gekennzeichnet*, daß** das Oligonucleotid
(i) als DNA oder
(ii) als (i) entsprechende RNA oder
(iii) als PNA vorliegt,
wobei das Oligonucleotid gegebenenfalls in einer für analytische Nachweisverfahren, insbesondere auf Basis von Hybridisierung und/oder Amplifizierung, an sich bekannten Weise modifiziert ist.

7. Kit nach Anspruch 6, **dadurch *gekennzeichnet,* daß** das Oligonucleotid **dadurch** modifiziert ist, daß bis zu 10 % der Nucleotide, insbesondere 1 oder 2 Nucleotide, durch für Sonden und/oder Primer an sich bekannte analoge Bausteine ersetzt sind, insbesondere durch Nucleotide, die bei Bakterien nicht natürlich vorkommen.

8. Kit nach Anspruch 6 oder 7 **dadurch *gekennzeichnet,* daß** das Oligonucleotid **dadurch** oder zusätzlich **dadurch** modifiziert bzw. markiert ist, daß es in einer für analytische Nachweisverfahren an sich bekannten Weise ein oder mehrere radioaktive Gruppen, farbige Gruppen, fluoreszierende Gruppen. Gruppen zur Immobilisierung an fester Phase und/oder Gruppen für eine indirekte oder direkte Reaktion, insbesondere eine enzymatische Reaktion, aufweist, insbesondere mit Hilfe von Antikörpern, Antigenen, Enzymen und/oder Substanzen mit Affinität zu Enzymen oder Enzymkomplexen, oder daß es in einer für analytische Nachweisverfahren an sich bekannten Weise andersartig modifizierte bzw. modifizierende Gruppen aufweist.

9. Verwendung eines Kits gemäß einem der Ansprüche 1 bis 8 zum Nachweis dit An- oder Abwesenheit von S. aureus **dadurch *gekennzeichnet,* daß** man eine Nucleinsäurehybridisierung und/oder eine Nucleinsäureamplifikation durchführt.

10. Verwendung nach Anspruch 9, **dadurch *gekennzeichnet,* daß** man eine Polymerase-Kettenreaktion als Nucleinsäureamplifikation durchführt.

## Claims

1. Kit comprising a plurality of oligonucleotides as primers and/or probes for the analytical detection of Staphylococcus aureus strains, **characterised by**
(a) an oligonucleotide which contains at least 10 successive nucleotides of SEQ ID NO 1 or their complementary nucleotides, and
(b) an oligonucleotide which contains at least 10 successive nucleotides of the region from
(i) nucleotide position 100 to 166 of SEQ ID NO 1 or
(ii) the sequence complementary to (i).

2. Kit according to claim 1 comprising an oligonucleotide according to (a) having a sequence that is shorter than SEQ ID NO 1, namely
(i) a sequence of the region or in the region of the nucleotide positions 54 to 83, or
(ii) a sequence of the region of the nucleotide positions 110 to 121, or
(iii) a sequence of the region of the nucleotide positions 127 to 156, or
(iv) a sequence that is complementary to a sequence according to (i), (ii) or (iii).

3. Kit according to claim 1 comprising an oligonucleotide having a sequence that is shorter than SEQ ID NO 1, namely
(i) SEQ ID NO 2, or
(ii) SEQ ID NO 3, or
(iii) SEQ ID NO 4, or
(iv) the sequences complementary to (i), (ii) and (iii), respectively.

4. Kit according to claim 1 comprising an oligonucleotide that is ***characterised* in that**, in respect of its sequence in at least 10 successive nucleotides of its nucleotide chain,
(i) it is identical to an oligonucleotide according to one of the preceding claims 2 or 3, or
(ii) it corresponds in 9 out of 10 successive nucleotides to an oligonucleotide according to one of the preceding claims 2 or 3, or
(iii) it corresponds in 8 out of 10 successive nucleotides to an oligonucleotide according to one of the preceding claims 2 or 3.

5. Kit according to claim 4, ***characterised* in that** the oligonucleotide is from 10 to 250 and preferably from 15 to 30 nucleotides long, especially **characterised in that** it is the nucleic acid molecule having the sequence SEQ ID NO 5.

6. Kit according to one of the preceding claims, ***characterised* in that** the oligonucleotide is present
(i) as DNA, or
(ii) as RNA corresponding to (i), or
(iii) as PNA,
the oligonucleotide, where appropriate, being modified in a manner known *per se* for analytical detection methods, especially methods based on hybridisation and/or amplification.

7. Kit according to claim 6, ***characterised* in that** the oligonucleotide is modified by the replacement of up to 10 % of the nucleotides, especially 1 or 2 nucleotides, by analogous components known *per se* for probes and/or primers, especially by nucleotides that do not occur naturally in bacteria.

8. Kit according to claim 6 or 7, ***characterised* in that** the oligonucleotide is modified or labelled or is additionally modified or labelled **in that** it comprises, in a manner known *per se* for analytical detection methods, one or more radioactive groups, coloured groups, fluorescent groups, groups for immobilisation on a solid phase and/or groups for an indirect or direct reaction, especially an enzymatic reaction, especially using antibodies, antigens, enzymes and/or substances having an affinity for enzymes or enzyme complexes, or it comprises, in a manner known *per se* for analytical detection methods, groups that have been modified or that modify in some other manner.

9. Use of a kit according to one of claims 1 to 8 for the detection of the presence or absence of S. aureus, ***characterised* in that** nucleic acid hybridisation and/or nucleic acid amplification is carried out.

10. Use according to claim 9, ***characterised* in that** a polymerase chain reaction is carried out as nucleic acid amplification.

## Revendications

1. Trousse comportant plusieurs oligonucléotides comme amorces et/ou sondes pour la détection analytique de souches de *Staphylococcus aureus,* **caractérisée par**
(a) un oligonucléotide qui contient au moins 10 nucléotides consécutifs de SEQ ID N°: 1 ou les nucléotides complémentaires de ceux-ci, et
(b) un oligonucléotide qui contient au moins 10 nucléotides consécutifs du domaine de
(i) la position de nucléotides 100 à 166 de SEQ ID N° : 1 ou
(ii) la séquence complémentaire de (i).

2. Trousse selon la revendication 1, comportant un oligonucléotide selon le point (a) avec une séquence réduite par rapport à SEQ ID N° : 1, à savoir
(i) une séquence du domaine ou dans le domaine des positions de nucléotides 54 à 83 ou
(ii) une séquence du domaine des positions de nucléotides 110 à 121 ou
(iii) une séquence du domaine des positions de nucléotides 127 à 156 ou
(iv) une séquence qui est complémentaires à une séquence selon (i), (ii) ou (iii).

3. Trousse selon la revendication 1, comportant un oligonucléotide présentant une séquence réduite par rapport à SEQ ID N° : 1, à savoir,
(i) la SEQ ID N° : 2 ou
(ii) lae SEQ ID N° : 3 ou
(iii) la SEQ ID N° 4 ou
(iv) la séquence complémentaire respectivement de (i), (ii), ou (iii).

4. Trousse selon la revendication 1, comportant un oligonucléotide, **caractérisée en ce que**, eu égard à sa séquence, pour au moins 10 nucléotides consécutifs de sa chaîne de nucléotides
(i) il est identique à un oligonucléotide selon l'une quelconque des revendications 2 ou 3 précédentes, ou
(ii) il correspond dans 9 nucléotides consécutifs sur 10, à un oligonucléotide selon l'une quelconque des revendications précédentes 2 ou 3, ou
(iii) il correspond dans 8 nucléotides consécutifs sur 10, à un oligonucléotide selon l'une quelconque des revendications 2 ou 3.

5. Trousse selon la revendication 4, **caractérisée en ce que** l'oligonucléotide présente une longueur de 10 à 250 nucléotides et de préférence, de 15 à 30 nucléotides, en particulier, **caractérisée en ce qu'**il est la molécule d'acide nucléique de la séquence SEQ ID N°: 5.

6. Trousse selon l'une quelconque des revendications précédentes, **caractérisée en ce que** l'oligonucléotide se présente
(i) sous forme d'ADN ou
(ii) sous forme d'ARN correspondant à (i) ou
(iii) sous forme de PNA,
l'oligonucléotide étant modifié éventuellement de façon connue en tant que telle pour des procédés de détection analytique, en particulier, basés sur l'hybridation et/ou l'amplification.

7. Trousse selon la revendication 6, **caractérisée en ce que** l'oligonucléotide est modifié **en ce que** jusqu'à 10 % de nucléotides, en particulier, 1 ou 2 nucléotides sont remplacés par des éléments analogues connus de sondes et/ou amorces, qui ne sont pas présents à l'état naturel dans les bactéries.

8. Trousse selon la revendication 6 ou 7, **caractérisée en ce que** l'oligonucléotide est, ou est en outre, modifié ou marqué, **en ce qu'**il présente de manière connue en tant que telle pour les procédés de détection analytique, un ou plusieurs groupes radioactifs, groupes colorés, groupes fluorescents, groupes d'immobilisation sur une phase solide et/ou groupes pour une réaction indirecte ou directe, en particulier, une réaction enzymatique, en particulier, à l'aide d'anticorps, d'antigènes, d'enzymes et/ou de substances présentant une affinité pour les enzymes ou complexes enzymatiques, et ou **en ce qu'**il présente de manière connue en tant que telle pour les procédés de détection analytique, des groupes modifiés ou modifiants d'une autre façon.

9. Utilisation d'une trousse selon l'une quelconque des revendications 1 à 8, pour la détection de la présence ou de l'absence de *S*. *aureus,* **caractérisée en ce que** l'on réalise une hybridation d'acide nucléique et/ou une amplification d'acide nucléique.

10. Utilisation selon la revendication 9, **caractérisée en ce que** l'on réalise une réaction en chaîne polymérase comme amplification d'acide nucléique.
